# EUROPEAN PATENT SPECIFICATION

(11) **EP 1 064 556 B1**
(45) Date of publication and mention of the grant of the patent: **23.11.2005**
(21) Application number: 99912169.2
(22) Date of filing: 29.03.1999
(51) Int. Cl.: G01N 33/80

(54) **SOLID-PHASE METHOD FOR ANTIGEN AND ANTIBODY DETERMINATIONS IN BLOODGROUP SEROLOGY, AND TEST KIT**
FESTPHASENVERFAHREN UND TESTSATZ ZUR ANTIGEN- UND ANTIKÖRPERPRÜFUNG IN DER BLUTGRUPPEN-SEROLOGIE
METHODE EN PHASE SOLIDE POUR DETERMINATIONS D'ANTIGENES ET D'ANTICORPS EN SEROLOGIE DES GROUPES SANGUINS ET KIT DE TEST

(30) Priority: 27.03.1998 NL 1008738
(43) Date of publication of application: 03.01.2001
(73) Proprietor: Stichting Sanquin Bloedvoorziening, 1066 CX Amsterdam (NL)
(72) Inventor: VAN DER DONK, Eric, Marinus, Maria, NL-1181 BD Amstelveen (NL); DEN BOER, Pieter, Johannes, NL-2313 KH Leiden (NL); VAN EIJK, Ronald, Victor, Wilhelmus, NL-3981 GM Bunnik (NL)
(74) Representative: Prins, Adrianus Willem
(86) International application number: PCT/NL1999/000181
(87) International publication number: WO 1999/050673

(56) References cited:
- EP-A- 0 363 510
- WO-A-95/30904
- WO-A-95/31731
- WO-A-98/02752
- US-A- 4 925 786

## Description

### Field of the invention

The invention lies in the field of bloodgroup serology and relates more particularly to a method and test kit for determining bloodgroup antigens, or antibodies directed thereto, in a sample.

### Background of the invention

The purpose of bloodgroup assays and antibody examination in clinical work is often to obtain compatible erythrocyte preparations for transfusion. The invention concerns the detection and identification of antibodies and antigens and can be used for bloodgroup assay, antibody screening and identification, and performing cross tests. The test is not based on hemagglutination but on the solid-phase principle, which does not involve washing steps.

Blood transfusion reactions which are induced by allo-antibodies to erythrocytes are called hemolytic transfusion reactions because they are mostly accompanied by an often very strongly accelerated breakdown of erythrocytes. The point is therefore to prevent hemolytic transfusion reactions by careful bloodgroup examination.

Bloodgroup antibodies are nearly always immunoglobulins of the IgG or IgM type. The antigen-antibody interaction is dependent *inter alia* on ionic bonding, hydrogen bridges and hydrophobic effects (displacement of water). The strength of a binding between a binding site of an antibody and an epitope is designated as 'affinity'. Antibodies which are able to agglutinate erythrocytes under all conditions are called agglutinins or complete antibodies (mostly IgM). Antibodies that bind to erythrocytes but do not give agglutination (sensitization) are called incomplete antibodies (mostly IgG).

There are a large variety of serological tests. The most important techniques at present are the tube method, the column test and tests in microtiter plates. A distinction can be made between techniques that are based on hemagglutination and techniques that are based on the solid-phase principle.

### a. Tests based on agglutination:

The tube test is a widely used test, where also prolonged incubations with antibodies are possible. The erythrocytes, after the reaction with antibodies, can be sedimented or be centrifuged to accelerate the agglutination reaction. An important and widely applied test is the antiglobulin or Coombs test. The antiglobulin test is based on the principle that erythrocytes loaded with, for instance, antibodies of the IgG type can be agglutinated by antiglobulin serum. In the test, three phases can be distinguished. The first phase is the sensitization phase. During this phase, antibodies bind to the corresponding antigen structures on the erythrocytes (sensitization of erythrocytes). When the binding is optimal, the second phase, viz. the washing phase, takes place. In this phase, all non-bound antibodies are removed from the incubation mixture. Insufficient removal of non-bound antibodies can lead to inactivation of the antiglobulin serum in that these antibodies bind to the antiglobulin. The third phase is the antiglobulin phase, in which antiglobulin is added to the washed sensitized cells, so that the sensitized cells are coupled to each other (agglutination of the erythrocytes).

A further simplification of serological tests has been carried through by centrifuging the erythrocytes through a column of (Sephadex) gel or glass beads. The use of transparent, inert, solid particles for distinguishing agglutinates and non-agglutinates was already described by Dalton et al. in 1970 (Becton, Dickinson & Co. US Patent 3,492,396). The system whereby gel material is used for the detection of erythrocyte-antibody reactions has been described by LaPierre et al. (Transfusion 1990; 30: 109-113, European Patents 0 194 212 and 0 305 337). In this test system, use is made of small columns filled with Sephadex gel. Use can be made of columns not containing any antibodies (e.g. for reverse ABO typing). As a second possibility, a gel column can also contain antibodies which are directed to certain erythrocyte antigens (e.g. for typing). For the antiglobulin test, use is made of gel columns which contain antiglobulin serum. After an incubation, the gel columns are centrifuged. In the case of a negative reaction, all erythrocytes will end up at the bottom of the tube; if the test is positive, the erythrocyte agglutinates will be captured on top of or in the gel. In the case of weak reactions, erythrocytes will sediment partly.

A major advantage of this test is that in case of the antiglobulin test no washing steps are needed anymore, since during the centrifugation separation of erythrocytes and serum or plasma components takes place. A second advantage is the fact that the results of the test can be properly fixed. The test described is used by DiaMed AG (DiaMed-ID Microtyping System).

A disadvantage of this test is that often problems occur in demonstrating weak agglutinates, for instance in the case of weak AB0-incompatibilities (Cummins & Downham, Lancet 1994; 343:1649 and Philips et al., Transfusion Medicine 1997; 7:47-53), possibly in that so-called "shear forces" during the centrifugation cause larger agglutinates to disintegrate into several small agglutinates or, in an extreme case, into individual (sensitized) erythrocytes. In spite of the sieve action of the gel, only weak to negative reactions are observed then, resulting in an increase of the number of false-negative reactions.

### b. Tests based on the solid-phase principle:

Another approach is the use of the solid-phase principle as an alternative to direct and indirect agglutination reactions for bloodgroup assay, antibody screening, antibody identification and cross tests. Application and advantages of the use of solid-phase techniques in the areas mentioned have been described by Rosenfield (Abstracts, 15th Cong. Int. Soc. Blood Trans., Paris pp. 27-33, 1976; US Patent 4,275,053, 1981). Here, inter alia erythrocytes were used which had been coupled to the surface of plastic tubes. More recently, systems have been described by Plapp et al. (Am. J. Clin. Path. 82: 719-721, 1984), Bayer et al. (US Patent 4,608,246, 1986), Rachel et al. (Transfusion 25: 24-26, 1985), Plapp et al. (The Lancet 1465-1466, 1986) and Uthemann et al. (US Patent 4,925,786, EP 363 510 and Transfusion 30: 114-116, 1990). Microtiter plates in combination with the solid-phase principle are used by, among others, Biotest AG (Solidscreen II for antibody diagnostics), Immucor Inc. (Immunocapture Capture-R systems for antibody screening and identification) and CLB (Microtype for typing erythrocyte antigens) and have further been described by Llopis et al. (Vox Sanguinis 1996; 70:152-156 and Vox Sanguinis 1997; 72:26-30), Ohgama et al. (Transfusion Medicine 1996; 6:351-359), Chateau et al. (La Gazette de la Transfusion 1997; 131:38-41, FR 94 05123) and Den Boer et al. (WO 98/02752).

The solid-phase tests are not limited to microtiter plates. Pernell (Sanofi Pasteur, EP 0 594 506) describes an affinity gel test in which an immunoglobulin-binding substance (such as protein A, protein G or anti-human IgG) is immobilized on the gel. Incubation of erythrocytes and antibodies occurs above the gel. After this incubation phase, centrifugation of the gel column takes place. If antibodies are bound to the erythrocytes, these sensitized erythrocytes will bind to the immunoglobulin-binding gel material and will hence bind to the upper part of the gel column. Cells to which no antibodies are bound will not be bound and end up at the bottom of the column. This principle of binding of sensitized cells to a solid phase has previously been described by Pharmacia (Cell Affinity Chromatography; Uppsala, Sweden; 1984). Pharmacia describes therein the purification of erythrocytes on the basis of the presence or absence of certain bloodgroup antigens using protein A-sepharose 6MB. Erythrocytes with the A antigen on the surface (bloodgroup A erythrocytes) to which anti-A antibodies are bound, can be separated by binding to said gel material (via protein A) from erythrocytes that do not possess the A antigen. The test described by Sanofi Pasteur is therefore a combination of this principle and the centrifugation step for separating erythrocytes, whether or not sensitized, and non-bound antibodies as used, for instance, in the column system of DiaMed.

A disadvantage of the above-described affinity gel test, where the ligands described are protein A, protein G or an antiglobulin, is that no complement factors are bound. Some antibodies are complement-binding (e.g. anti-Jk antibodies): the binding of a single antibody molecule to a particular erythrocyte antigen leads to the binding of many complement molecules on the erythrocyte membrane. Consequently, many allo-antibodies, for instance, can be demonstrated with much higher sensitivity with anti-complement than with anti-immunoglobulin antibodies. In fact, many examples have been described of antibodies that could be demonstrated exclusively with anti-complement (Mollison et al., Blood Transfusion in Clinical Medicine, Blackwell Scientific Publications 1992). Accordingly, tests that do not display anti-complement activity involve a chance of false-negative reactions.

The disadvantage of the use of protein A as immunoglobulin-binding material is that antibodies of the type IgG3 cannot be demonstrated (the fact is that protein A only binds the IgG subclasses IgG1, IgG2 and IgG4), although these may be clinically relevant. IgM antibodies, which are also clinically relevant, are alleged not to be bound by protein G and only partly by protein A.

### Brief summary of the invention

The invention now provides a method for determining in a sample an analyte selected from a bloodgroup antigen present on erythrocytes or an antibody binding to such a bloodgroup antigen,
comprising treatment of the sample in an incubation zone of a reaction vessel with a reagent containing an analyte-binding partner, which analyte-binding partner, in the case where the analyte is a bloodgroup antigen present on erythrocytes, is an antibody capable of binding to the bloodgroup antigen, and in the case where the analyte is an antibody binding to a bloodgroup antigen, is the bloodgroup antigen present on erythrocytes, wherein in the incubation zone, if the sample contains the analyte, either a complex is formed of bloodgroup antigen present on erythrocytes and antibody bound thereto, or a complex is formed of bloodgroup antigen present on erythrocytes and antibody bound thereto, as well as a complex of complement factors bound to these erythrocytes if the antibody is complement-binding,
further comprising separation of erythrocytes, complexed or not, from non-bound antibodies using a separation medium, located in the reaction vessel under the incubation zone, of a density higher than that of the fluid containing the antibodies but lower than the density of erythrocytes, whereby erythrocytes pass through the separation medium and non-bound antibodies remain in the incubation zone,
separation of complexed erythrocytes from non-complexed erythrocytes by binding complexed erythrocytes in an immobilization zone of the reaction vessel wherein the immobilization zone comprises a gel matrix carrying IgM binding antibody, bound to the gel matrix by direct covalent binding or via a spacer arm, discharging non-complexed erythrocytes to a collection.,zone of the reaction vessel, and
detection of erythrocytes in the immobilization zone and/or collection zone.

The present invention also provides a test kit suitable for use in a method for determining an analyte in a sample, the analyte being a bloodgroup antigen present on erythrocytes or an antibody binding to such a bloodgroup antigen, comprising:
(i) a reagent containing an analyte-binding partner which, in the case where the analyte is a bloodgroup antigen present on erythrocytes, is an antibody capable of binding to the bloodgroup antigen, and in the case where the analyte is an antibody binding to a bloodgroup antigen, is the bloodgroup antigen present on erythrocytes,
(ii) a reaction vessel comprising an incubation zone, an immobilization zone and a collection zone, wherein the immobilization zone comprises a gel matrix carrying IgM-binding antibody or antibody fragment bound to the gel matrix by direct covalent coupling or via a spacer arm and optionally further carrying complement-binding antibody or antibody fragment bound to the gel matrix by direct covalent coupling or via a spacer arm,
(iii) a separation medium having a density lower than the density of erythrocytes but higher than the density of an antibody-containing fluid.

### Brief description of the drawings

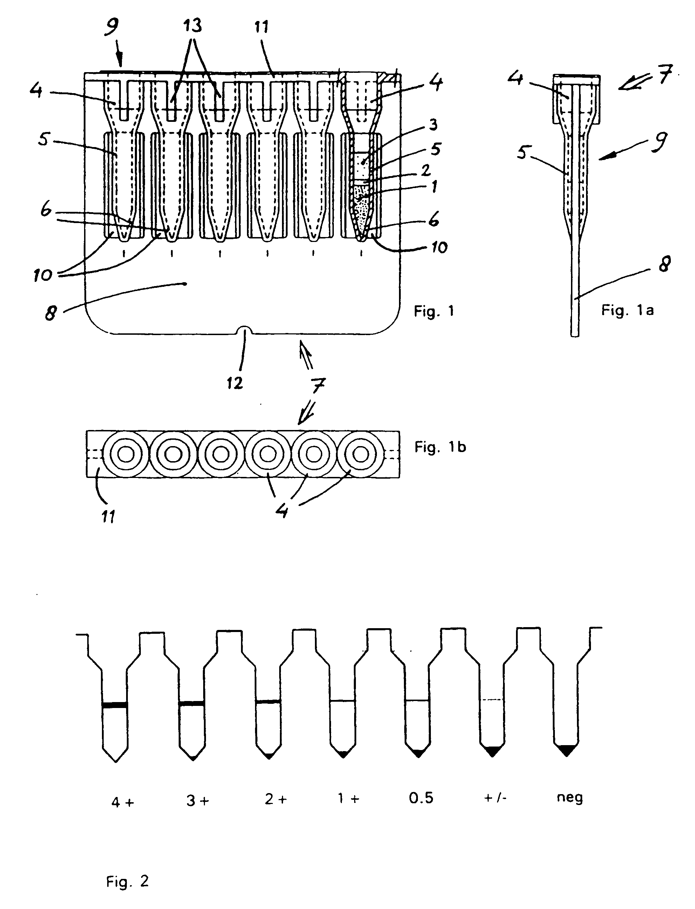

Fig. 1: this figure shows a test card with six microcolumns on it. In a microcolumn (9) closed at the bottom, a matrix of inert particles (1) is provided to support a thin layer of affinity gel matrix (2). On this affinity gel matrix there is a layer of separation medium of high density (3). The incubation compartment (4) is formed by a widening at the top of the microcolumn. A microcolumn test system (7) can be integrated into a base plate (8) and be placed in a centrifuge using supporting and guiding elements (11, 12, 13).
   The reference numerals denote:
   - 1: matrix of inert particles
   - 2: affinity gel matrix
   - 3: separation medium of high density
   - 4: incubation compartment
   - 5: microcolumn wall
   - 6: microcolumn bottom
   - 7: test card
   - 8: base plate
   - 9: microcolumn
   - 10: recess
   - 11: top surface
   - 12: slot
   - 13: support
Fig. 2: this figure shows the various possible reaction strengths. How many sensitized erythrocytes will bind to the affinity gel matrix depends on various parameters, such as the antigen density on the erythrocytes, the titer of the antibody in question, and the affinity of the antibody for the erythrocyte antigen in question. In general, in reactions weaker than 4+, erythrocytes will also be found at the bottom of the microcolumn.
   The strength indications denote:
   - 4+: all cells bound to the affinity matrix, no cells at the bottom
   - 3+: more cells bound to the affinity matrix than at the bottom
   - 2+: as many cells bound to the affinity matrix as at the bottom
   - 1+: fewer cells bound to the affinity matrix than at the bottom
   - 0.5: few cells bound to the affinity matrix, many cells at the bottom
   - +/-: hardly any cells bound to the affinity matrix, nearly all cells at the bottom
   - neg: no cells bound to the affinity matrix, all cells at the bottom

### Detailed description of the invention

The invention relates to a method for demonstrating antibodies and antigens, the application of this method lying mainly in the field of bloodgroup serology: bloodgroup determination, antibody screening and antibody identification, and cross tests.

The invention relates to an improvement of the solid-phase test as described by Pernell (Sanofi Pasteur, EP 0 594 506). A disadvantage of this affinity gel test of Sanofi Pasteur, where an immunoglobulin-binding substance (such as protein A, protein G or anti-human IgG) is immobilized on the gel matrix, is that no complement factors are bound and that IgM-antibodies are not bound or only partly so. In serology, however, in addition to IgG-antibodies, IgM-antibodies are also relevant, as are erythrocytes which are loaded with complement factors (for instance, in demonstrating complement-binding antibodies such as anti-Jk antibodies, also referred to as complement-dependent antibodies). To be able to also demonstrate these IgM-antibodies and/or complement-dependent antibodies with a high sensitivity, in the present invention, in addition to IgG-binding substances also IgM-binding and optionally complement-binding antibodies or antibody fragments are immobilized on the gel matrix, which leads to a lower number of false-negative reactions. The invention further encompasses the possibility that, immobilized on the gel matrix, an IgM-binding antibody or antibody fragment alone, without IgG-binding substance, is utilized.

According to the invention, sensitized erythrocytes can be bound directly to this affinity matrix without intervention of an anti-globulin serum.

Above the affinity matrix there is a fluid medium having a density between the density of erythrocytes and the density of the antibody-containing fluid. The presence of this layer provides that no separate washing step is needed to separate non-bound antibodies from the erythrocytes sensitized or not. Above this layer, incubation of erythrocytes and antibodies takes place. After the incubation phase, a centrifugation phase takes place, such that the erythrocytes are centrifuged through-the high-density medium. The antibody-containing fluid remains above this high-density medium. In this manner, therefore, the erythrocytes are separated from non-bound antibodies, coming, for instance, from the serum or plasma. If complement factors and/or IgG antibodies and/or IgM antibodies are bound to the erythrocytes, these sensitized erythrocytes will bind to the affinity matrix and so will bind to the upper part of the gel column. Cells which have not been sensitized will not be bound to the affinity matrix and can readily pass it.

According to the invention, it has been found to be of essential importance that in addition to the optional IgG-binding substance, complement-binding substance and/or IgM-binding substance be covalently immobilized on the solid phase and not be added loosely to the solid phase, as is the case, for instance, in the agglutination test described by Lapierre. Using a combination of IgG-binding, IgM-binding and complement-binding substances covalently immobilized on the gel matrix, an unexpected positive result was achieved. Firstly, it was found possible to demonstrate, in addition to IgG antibodies, complement-dependent antibodies with a high sensitivity. In addition, it also proved possible to demonstrate agglutinins (bloodgroup antibodies of the IgM type, of importance in performing cross tests with serum from the recipient and erythrocytes from the donor) with a high sensitivity, including weak agglutinins.

Accordingly, the invention provides a composite solid phase and a method for determining in a sample an analyte selected from a bloodgroup antigen present on erythrocytes or an IgG antibody and/or IgM antibody and/or complement-dependent antibody binding to such a bloodgroup antigen.

The invention further provides a test kit comprising:
(i) a reagent containing an analyte-binding partner which, in the case where the analyte is a bloodgroup antigen present on erythrocytes, is an antibody capable of binding to the bloodgroup antigen, and in the case where the analyte is an IgG antibody and/or IgM antibody and/or complement-dependent antibody binding to a bloodgroup antigen, is the bloodgroup antigen present on erythrocytes,
(ii) a reaction vessel comprising an incubation zone, an immobilization zone and a collection zone, wherein said immobilization zone comprises a gel matrix carrying IgM antibody or antibody fragment bound to the gel matrix by direct covalent coupling or via a spacer arm and optionally complement-binding antibody or antibody fragment bound to the gel matrix by direct covalent binding a via a spacer arm, and
(iii) a separation medium having a density lower than the density of erythrocytes but higher than the density of an antibody-containing fluid.

The invention described here has as an advantage over existing techniques that the sensitivity of the test, owing to the combination used of the IgG-binding, IgM-binding and complement-binding substances immobilized on the gel matrix, is unexpectedly high. Both agglutinins, including weak agglutinins, and complement-dependent antibodies can be demonstrated with a highly increased sensitivity over the conventional microcolumn tests. Accordingly, this leads to a lower number of false-negative reactions.

The invention described can be applied to the current solid-phase tests within bloodgroup serology, including screen tests for antibodies, identification of antibodies, cross-tests, antigen determination, bloodgroup determination and antibody titration. The invention can be used, for instance, for determining the following bloodgroup antigens and antibodies to those antigens: ABO, Kell (K), cellano (k), Duffy (Fy^{a} and Fy^{b}), Kidd (Jk^{a} and Jk^{b}), Lutheran (Lu^{a} and Lu^{b}), the Rhesus system (D, E, e, C, c), MNS, Lewis (Le^{a} and Le^{b}), *et cetera.*

By way of example, the invention can be used for the following assays:
- Tests in which use is made of an antiserum of a known specificity and erythrocytes of unknown antigen composition (bloodgroup assay, antigen assay).
- Tests in which use is made of sera having therein an unknown antibody or unknown antibodies and erythrocytes having a known antigen composition (antibody screening, antibody identification, allo-antibody assay (also referred to as reverse grouping). Here, often use is made of' (a panel of) erythrocytes of known antigen composition. In addition, the test can also be used for the detection of auto-antibodies. In this case, antibodies are already bound to the corresponding antigens on the erythrocytes, so the erythrocytes are already sensitized and can bind directly to the composite solid phase.
- Tests utilizing serum from the recipient and erythrocytes from the donor to demonstrate any antibodies in the serum from the recipient (cross test).
- Tests performed in accordance with the present invention are all based on the same principle: erythrocytes to which antibodies are bound (sensitized erythrocytes) can bind to the composite solid phase, in contrast with non-sensitized erythrocytes.

The sample will typically consist of blood or a material derived therefrom, such as blood plasma, blood serum or a blood fraction, for instance erythrocytes isolated from blood. However, since the invention can also be used for other purposes, such as selecting hybridomas which produce monoclonal antibodies that bind to a bloodgroup antigen, the sample can also consist of materials of a different kind, for instance of a supernatant of a hybridoma.

It is essential that during the incubation of antibodies with erythrocytes and the centrifugation step there be no contact between this incubation mixture and the composite solid phase, for which reason a medium of high density has been applied to the composite solid phase. Additionally of essential importance are: the choice of immunoglobulin-binding and complement-binding substances, the choice of the material to increase the density of the medium, and the choice of gel matrix material. These points will be briefly explained here.
a. Immunoglobulin-binding substances.
   The choice from immunoglobulin-binding substances that can be immobilized on the solid phase is wide. Useful are, for instance, polyclonal or monoclonal antibodies directed to immunoglobulin G (IgG), immunoglobulin A (IgA) and/or immunoglobulin M (IgM), or the Fab or F(ab)'₂ fragments thereof. In order to increase the sensitivity, or to detect a widest possible range of antibodies, it is also possible to immobilize a combination of several immunoglobulin-binding substances on the solid phase.
   It is also possible to use immunoglobulin-binding substances of a different origin, for instance from bacteria. Immunoglobulin-binding bacterial proteins are used, for instance, in immunology; biochemistry, and biotechnology. A number of immunoglobulin-binding proteins have been isolated and characterized.
   The most important are protein A (Forsgren et al. J. Immunol. 1966; 97: 822) and protein G (Björck et al. J. Immunol. 1984; 133: 969; Reis et al. J. Immunol. 132: 3091).
   For the tests involving IgG antibodies (antibody identification, antibody screening and cross tests), use can be made of a solid phase with an IgG-binding substance of a high affinity, so that optimum binding of sensitized cells to a solid phase is ensured. As immunoglobulin-binding substance, in addition to anti-human immunoglobulin, again protein G can be used. A solid phase on which both IgG-binding substances and IgM-binding substances are immobilized is preferred, because in that case a wider range of antibody specificities can be demonstrated. Any IgA-alloantibodies are, normally speaking, always accompanied by IgG antibodies of the same specificity.
b. Complement-binding substances.
   When complement is activated after the binding of complement-binding antibodies to an erythrocyte antigen, the activation products of the complement factors C4 and especially C3, C4b and C3b, respectively, are bound on the erythrocyte membrane. Accordingly, useful as complement-binding substances are, for instance, polyclonal or monoclonal antibodies directed against antigenic determinants on relevant complement factors, such as, for instance, C3c, C3d and C3g on the complement factor C3b, or combinations hereof.
c. Substances for increasing the density of the medium can be many kinds of substances, including, e.g., albumin, dextran, Ficoll, iodixanol, sodium diatrizoate, Percoll, etc. The density of the medium should be higher than that of the antibody-containing fluid, but lower than that of erythrocytes, so the density must lie between 1.01 and 1.09 g/ml, preferably between 1.02 and 1.06 g/ml.
d. Gel matrix material.

Suitable as matrix material are, for instance, agarose (e.g. Sepharose 4 Fast Flow, Sepharose High Performance (Pharmacia)) or acrylic resin. Also of importance is the size of the particles: in a matrix with larger particles, and hence more interspace, the binding of sensitized erythrocytes will proceed slowly, so that insufficient cells can be bound. A finer matrix will lead to a more intensive contact of the sensitized cells with the immunoglobulin-binding and complement-binding substances, so that optimum binding of the cells is possible. Too fine a matrix, however, will lead to clogging. The diameter of the particles will therefore have to lie between 10 and 125 µm, preferably between 20 and 50 µm.

The immunoglobulin-binding and complement-binding substances are immobilized on the solid phase through direct covalent binding, or via a spacer arm. Such a spacer arm can have a chain length of, for instance, 5-10 carbon atoms and has as an advantage that the binding substances (ligands) become better accessible to sensitized erythrocytes in that steric hindrance is reduced. In the use of NHS-activated Sepharose (Pharmacia) as solid phase, for instance, a spacer arm is already present on this matrix material; when now a ligand with primary amino groups is presented, there arises, with cleavage of N-hydroxysuccinimide from the activated ester compound, a solid phase to which ligands are bound via a spacer of 6 carbon atoms (i.e. a bridge of a chain length of 6 C atoms). It is also possible, however, first to immobilize, for instance, protein G on the gel matrix and then to couple anti-human complement and/or anti-human IgM to protein G itself, either directly or through so-called ligand bridges, for instance bridge-forming antibodies, which are first coupled to the protein G and are directed to human antibodies.

For carrying out the test, a test system is preferred which consists of a card with microcolumns manufactured from a transparent inert plastic, allowing single and multiple tests to be performed (see also Fig. 1). In these microcolumns, a matrix of inert particles is provided (e.g. glass beads (Supelco) or Streamline (quartz base matrix, Pharmacia)). This inert matrix serves to support a thin layer of gel matrix on which immunoglobulin-binding and complement-binding substances are immobilized. Provided on this affinity gel matrix is a layer of separation medium of a density higher than that of the antibody-containing fluid but lower than that of erythrocytes. This layer is of importance to the invention, since the presence of this layer eliminates the necessity of a separate washing step to separate non-bound antibodies from the (sensitized) erythrocytes. The incubation of erythrocytes and antibodies can take place in a compartment formed by a widening at the top of the microcolumn. After the incubation phase in which antibodies can bind to the cells, the erythrocytes, whether sensitized or not, are separated from non-bound antibodies by centrifugation through the separation medium mentioned, and transported to the affinity gel matrix, whereafter the sensitized cells will adhere to the affinity gel matrix, while the non-sensitized cells will be transported through the affinity gel matrix to the bottom of the microcolumn. The required centrifugation protocol, as regards centrifugal force (g-value) and duration, depends inter alia on the amount of erythrocytes to be used, the density of the separation medium mentioned, and the particle size of the gel matrix. It is possible, for instance, to opt for a so-called linear centrifugation protocol, whereby the g-value rises in a particular time period (for instance 1 min) to a given maximum value (e.g. 2000xg), which is maintained for a certain period of time (e.g. 9 min). As the g-value rises, the erythrocytes will be transported through the separation medium, allowing the sensitized erythrocytes to bind to the affinity matrix. Thereupon, during the period at maximum g-value, all non-sensitized erythrocytes will be transported through the affinity matrix to the bottom of the microcolumn. For carrying out the test, a so-called discontinuous centrifugation protocol is preferred, whereby during the 1st phase, at a particular g-value (e.g. 10-60 sec at 400-1000xg, preferably 20-40 sec at 400-500xg), erythrocytes will be transported through the separation medium, whereafter, in the 2^{nd} phase, at a lower g-value (e.g. 10-60 sec at 50-400xg, preferably 20-40 sec at 100-300xg), the sensitized erythrocytes can bind to the affinity matrix, whereafter during the last phase, at maximum g-value (e.g. 1-13 min at 1000-2500xg, preferably 7-10 min at 1800-2000xg) all non-sensitized erythrocytes are transported through the affinity matrix to the bottom of the microcolumn. It is of essential importance that all non-sensitized cells end up at the bottom, so as to prevent false-positive reactions, so centrifugation in the last phase should be relative long and at high speed.

Detection of the bound cells on the affinity gel matrix and non-bound cells at the bottom of the microcolumn can simply be done both visually and automatically. The various possible reaction patterns are shown in Fig. 2. How many sensitized erythrocytes will bind to the affinity gel matrix depends on various parameters, such as the antigen density on the erythrocytes, the titer of the antibody in question, and the affinity of the antibody for the erythrocyte antigen in question. In general, in reactions weaker than 4+, cells will also be found at the bottom of the microcolumn.

It has proved to be of essential importance for the invention that the immunoglobulin-binding substances and complement-binding substances be covalently immobilized on the solid phase and not be added loosely to the gel matrix, as is the case, for instance, in the column agglutination test described by Lapierre (EP 0 305 337). Preferably used as immunoglobulin-binding substances are protein G and antibodies directed against human IgM (anti-human IgM). As complement-binding substances, preferably antibodies are used which are directed against the human complement factor C3d (anti-human C3d). Using the combination of protein G, anti-human IgM and anti-human C3d immobilized on the gel matrix, an unexpected positive result was achieved:
Firstly, it proved possible to properly demonstrate complement-dependent antibodies. When, on the other hand, protein G alone was immobilized on the gel matrix, these complement-dependent antibodies were only weakly demonstrable. When immunoglobulin-binding substances and complement-binding substances were not immobilized but were added loosely to the gel matrix (in this case anti-human IgG and anti-human C3d, whereby in fact an indirect antiglobulin test was obtained, as described by Lapierre), the complement-dependent antibodies were hardly demonstrated, if at all.
Secondly, it also proved possible to properly demonstrate agglutinins:
   - bloodgroup antibodies of the IgM type, which still gave sensitization of the erythrocytes but no visible agglutination anymore in the tube test, were still properly demonstrable in the present test, in contrast to the situation where protein G alone was immobilized on the gel matrix. When anti-human IgM and anti-human C3d were added loosely to the gel matrix, the diluted IgM bloodgroup antibodies were hardly, if at all, demonstrable. When gel matrix alone, without ligands, was used, the diluted IgM bloodgroup antibodies were not demonstrable. This unexpected result could be explained by the fact that the present test is an affinity test, which is not dependent on the sieve action of the gel matrix for holding agglutinates, but wherein individual sensitized erythrocytes can still be bound to the gel matrix.
   - when weak AB0-incompatibilities were tested, e.g. A.B erythrocytes/B-piasma incompatibility, the reactions were properly demonstrable in the present test, in contrast to the situation where protein G alone was immobilized on the gel matrix. When anti-human IgM and anti-human C3d were added loosely to the gel matrix, the reactions were hardly demonstrable, if at all. When gel matrix alone, without ligands, was used, the reactions were not demonstrable. It is a well known given that "neutral" and antiglobulin microcolumn tests have problems in demonstrating weak AB0-incompatibilities (Cummins & Downham, Lancet 1994; 343:1649 and Philips et al., Transfusion Medicine 1997; 7:47-53), possibly in that so-called "shear forces" during centrifugation cause larger agglutinates to disintegrate into several small agglutinates or, in an extreme case, into individual (sensitized) erythrocytes. In spite of the screening action of the gel, only weak to negative reactions are observed then, resulting in an increase of the number of false-negative reactions. As a possible solution to this problem, in the article of Philips et al. the Diamed test is performed according to a modified, discontinuous centrifugation protocol: by lowering the centrifugal force and tripling the centrifugation time, the weak agglutinates now prove to be better demonstrable, however, this modification is qualified as unsuited for routine use. Now, therefore, the present invention has the advantage that smaller agglutinates and even individual sensitized erythrocytes can still be bound to the affinity matrix, so that this kind of weak reactions can still be demonstrated well.

For that matter, it proved possible to demonstrate the weak agglutinins described equally well with a combination of solely protein G and anti-human IgM, immobilized on the gel matrix, and also with anti-human. IgM alone, immobilized on the gel matrix.

Likewise, it proved possible to demonstrate the complement-dependent antibodies equally well with a combination of just protein G and anti-human C3d, immobilized on the gel matrix, and also with anti-human C3d alone, immobilized on the gel matrix.

When these last four variant embodiments are used in addition to the described variant with the immobilized combination of protein G, anti-human IgM and anti-human C3d, it can be discriminated whether a given sample contains IgG-antibodies and/or IgM-antibodies and/or complement-dependent antibodies.

### Examples

The invention will now be further explained in and by the following examples. It is of importance to see, however, that these examples are given for illustrative purposes and illuminate the enablement of the invention but do not in any way constitute the definitive conditions of the different assay methods nor limit the scope of the invention in any way.

### Example 1

Glass beads (Supelco) were washed twice in a phosphate-buffered saline solution (PBS) and included in a 20 mM phosphate buffer, further containing 70 mM NaCl, 20 mM EDTA, 30 mM glucose, 5% dextran, 1.5% BSA, 0.02% gelatin, pH 8.8 (gel buffer).

10 ml Sepharose HP having immobilized thereon protein G (binding capacity for human IgG: 20-30 mg/ml gel, Pharmacia, Uppsala, Sweden)) was washed according to the instructions of the supplier and included in a buffer containing 20 mM Tris and 150 mM NaCl. To this was added monoclonal mouse IgG, directed to human IgM, to a final concentration of 100 µg/ml gel and monoclonal mouse IgG, directed to human C3d, to a final concentration of 75 µg/ml gel. After an incubation period of 12 hours at 4°C the affinity gel was washed twice in gel buffer. After each washing step, the supernatant was checked for the presence of loose anti-human IgM and anti-human C3d: these components were not demonstrable, so that all added anti-human IgM and anti-human C3d had to be coupled to the protein G. This affinity gel was then included in 25 ml gel buffer. Per microcolumn there was added: 15 µl settled glass beads in gel buffer. To this was added: 15 µl affinity gel suspension in gel buffer (20%). This affinity gel sedimented within 15 min on top of the glass beads. The supernatant gel buffer functions as high-density separation medium.

Erythrocytes of bloodgroup 0⁺ (i.e. bloodgroup 0, rhesus D positive) were sensitized with anti-D and then washed 3x in a modified LISS (low ionic strength saline) solution. Of these erythrocytes, a 0.5% suspension was made in LISS. Then 1 drop of this suspension was introduced into the incubation compartment of the microcolumn. To this was added 1 drop of normal serum from a donor of bloodgroup 0⁺. After an incubation period of 15 min at 37°C the microcolumn was centrifuged according to the following protocol: 30 sec at 500xg, followed by 30 sec at 200xg, followed by 9 min at 1950xg. Then the result was assessed: all erythrocytes had been bound to the upper side of the affinity gel matrix (reaction strength 4+). As control, the procedure described was repeated with non-sensitized erythrocytes of bloodgroup 0⁺. These cells were not bound to the affinity gel matrix.

### Example 2

Use was made of the test system as described in Example 1.

Of Duffy-a (Fy^{d}) positive erythrocytes (phenotype Fy^{a+b+}) a 0.5% suspension in LISS was made. Then 1 drop of this suspension was introduced into the incubation compartment of the microcolumn. To this was added 1 drop of anti-Fy^{a} (polyclonal IgG antiserum, CLB, Amsterdam, Netherlands). After an incubation period of 15 min at 37°C the microcolumn was centrifuged according to the protocol of Example 1. After this, the result was assessed: all erythrocytes were bound to the top side of the affinity gel matrix (reaction strength 4+). As control experiment, corresponding non-sensitized erythrocytes were tested. These cells were not bound to the affinity gel matrix.

This experiment was repeated for the following antigens: Fy^{b} (with anti-Fy^{b} serum with a Fy^{a+b+} cell), Jk^{a} (with anti-Jk^{a} serum and Jkd^{a+b+} cell), Jk^{b} (with anti-Jk^{b} serum and Jk^{a+b+} cell), Kell (with anti-K serum and K⁺k⁺ cell), cellano (with anti-k serum and K⁺k⁺ cell), Lu^{a} (with anti-Lu^{a} serum and Lu^{a+b+} cell), Lu^{b} (with anti-Lu^{b} serum and Lu^{a-b+} cell), S (with anti-S serum and S⁺s⁺ cell) and s (with anti-s serum and S⁺s⁺ cell). The results were in agreement with what has been described for Fy^{a}. In all cases, complete binding of sensitized erythrocytes to the affinity gel matrix occurred, while non-sensitized erythrocytes in the control experiments were not bound to the affinity matrix.

In the examples below, use was made of the following test systems:
system 1: the test system as described in Example 1 having as immobilized ligands on the gel matrix: protein G, anti-human IgM and anti-human C3d.
system 2: as system 1, but with protein G alone as immobilized ligand on the gel matrix.
system 3: as system 1, but with anti-human IgM alone as immobilized ligand on the gel matrix. To that end, an optimum amount of anti-human IgM was coupled to NHS-activated Sepharose HP according to the instructions of the supplier (Pharmacia). After the coupling, any residual activated groups were inactivated and the affinity gel was washed twice in gel buffer. After each washing step, the supernatant was checked for the presence of loose anti-human IgM: this was not demonstrable, so that all added anti-human IgM had to be coupled to the gel matrix.
system 4: as system 1, but without immobilized ligands on the gel matrix. To that end, NHS-activated Sepharose HP was inactivated according to the instructions of the supplier (Pharmacia).
system 5: as system 4, except that subsequently per microcolumn optimum amounts of immunoglobulin-binding and complement-binding substances, anti-human IgG and anti-human C3d, respectively, were added loosely, such that this was homogeneously distributed over glass beads, gel matrix and supernatant gel buffer. This in fact yielded an anti-globulin test system.
system 6: as system 5, except that immediately prior to the deployment of the test the supernatant gel buffer having therein loose anti-human IgG and anti-human C3d was replaced with normal gel buffer, so that the loosely added anti-human IgG and anti-human C3d were disposed only in the section with gel matrix and glass beads.
system 7: as system 4, except that subsequently per microcolumn optimum amounts of immunoglobulin-binding and complement-binding substances, anti-human IgM and anti-human C3d, respectively, were added loosely, such that this was homogeneously distributed over glass beads, gel matrix and supernatant gel buffer. This in fact yielded an anti-globulin test system.
system 8: as system 7, except that immediately prior to the deployment of the test the supernatant gel buffer having therein loose anti-human IgM and anti-human C3d was replaced with normal gel buffer, so that the loosely added anti-human IgM and anti-human C3d were disposed only in the section with gel matrix and glass beads.

### Example 3

A number of the above-mentioned test systems were examined for their ability to demonstrate complement-dependent antibodies. For this purpose, two patient sera were tested which were known to contain complement-dependent anti-jk^{a} antibodies (IgG). Of Jk^{a+b-} cells, a 0.5% suspension in LISS was prepared. Then 1 drop of this suspension was introduced into the incubation compartment of the microcolumn. To this was added 1 drop of patient serum. As control, this experiment was carried out with AB-serum instead of patient serum. After an incubation period of 15 min at 37°C, the microcolumn was centrifuged according to the protocol of Example 1. After this, the result was assessed. The reaction strengths are summarized in Table 1:

**Table 1**

| | system 1 | system 2 | system 5 | system 6 |
|---|---|---|---|---|
| patient serum 1 | 3+ | 1+ | 0.5 | 0.5 |
| patient serum 2 | 1+ | +/- | -- | -- |
| AB-serum | -- | -- | -- | -- |

### Example 4

A number of the above-mentioned test systems were examined for their ability to demonstrate weak agglutinins. To that end, a human monoclonal anti-D antibody of the type IgM (anti-D pelikloon, CLB) was diluted in AB-serum, such that sensitization of 0⁺ test erythrocytes still occurred, but no visible agglutination occurred anymore in a normal tube agglutination test in a physiological salt solution. Of 0° test erythrocytes (CLB) a 0.5% suspension in LISS was prepared. Then 1 drop of this suspension was introduced into the incubation compartment of the microcolumn. To this was added 1 drop of diluted anti-D. As control, this experiment was carried out with AB-serum. After an incubation period of 15 min at 37°C, the microcolumn was centrifuged according to the protocol from Example 1. Then the result was assessed. The reaction strengths are listed in Table 2:

**Table 2**

| | syst 1 | syst 2 | syst 3 | syst 4 | syst 7 | syst 8 |
|---|---|---|---|---|---|---|
| anti-D, diluted | 2+ | 0.5 | 2+ | -- | -- | -- |
| AB-serum | -- | -- | -- | -- | -- | -- |

### Example 5

A number of the above-mentioned test systems were examined for their ability to demonstrate agglutinins. To that end, an AB0-incompatibility was tested, viz. an A₂B erythrocyte sample with a bloodgroup B plasma which in a normal agglutination test in a physiological salt solution still gave a 1+ reaction. Of the A₂B erythrocytes, a 0.5% suspension in LISS was prepared. Then 1 drop of this suspension was introduced into the incubation compartment of the microcolumn. To this was added 1 drop of the B plasma. As control, this experiment was carried out with A₂B plasma of the A₂B erythrocytes donor (autocontrol). After an incubation period of 15 min at 37°C, the microcolumn was centrifuged according to the protocol from Example 1. After this, the result was assessed. The reaction strengths are listed in Table 3:

**Table 3**

| | syst 1 | syst 2 | syst 3 | syst 4 | syst 7 | syst 8 |
|---|---|---|---|---|---|---|
| B plasma | 2+ | 0.5 | 2+ | -- | +/- | -- |
| A₂B plasma | -- | -- | -- | -- | -- | -- |

It appears from Tables 2 and 3 that with system 2 (protein G alone as immobilized ligand on the gel matrix) a reaction is obtained which, though less strong, is still positive. Since with a neutral gel without ligands (system 4) a negative reaction is obtained, this is apparently the result of binding of IgM-erythrocyte complexes to protein G.

## Claims

1. A method for determining in a sample an analyte, selected from a bloodgroup antigen present on erythrocytes or an antibody binding to such a bloodgroup antigen,
comprising treating the sample in an incubation zone of a reaction vessel with a reagent containing an analyte-binding partner, which analyte-binding partner, in the case where the analyte is a bloodgroup antigen present on erythrocytes, is an antibody capable of binding to the bloodgroup antigen, and in the case where the analyte is an antibody binding to a bloodgroup antigen, is the bloodgroup antigen present on erythrocytes, wherein, in the incubation zone, if the sample contains the analyte, either a complex of bloodgroup antigen present on erythrocytes and antibody bound thereto is formed, or a complex of bloodgroup antigen present on erythrocytes and antibody bound thereto is formed, as well as a complex of complement factors bound to these erythrocytes if the antibody is complement-binding,
further comprising separating erythrocytes, complexed or not, from non-bound antibodies using a separation medium, located in the reaction vessel under the incubation zone, of a density higher than that of the fluid containing the antibodies but lower than the density of erythrocytes, whereby erythrocytes pass through the separation medium and non-bound antibodies remain in the incubation zone,
separating complexed erythrocytes from non-complexed erythrocytes by binding complexed erythrocytes in an immobilization zone of the reaction vessel,
discharging non-complexed erythrocytes to a collection zone of the reaction vessel, and
detecting erythrocytes in the immobilization zone and/or collection zone,
wherein said immobilization zone comprises a gel matrix carrying IgM-binding antibody or antibody fragment, bound to the gel matrix by direct covalent binding or via a spacer-arm.

2. A method according to claim 1, **characterized in that** the sample consists of blood, blood plasma, blood serum, a blood fraction or a supernatant of a hybridoma.

3. A method according to one or more of claims 1-2, **characterized in that** the gel matrix further carries complement-binding antibody or antibody fragment, bound to the gel matrix by direct covalent binding or via a spacer-arm.

4. A method according to claim 3, **characterized in that** the complement-binding antibody or antibody fragment binds an activation product of complement factor C4 or complement factor C3.

5. A method according to claim 4, **characterized in that** an antibody directed against an antigenic determinant on C3b or C4b, e.g. against C3c, C3d or C3g on C3b is used.

6. A method according to one or more of claims 1-5, **characterized in that** the spacer arm contains a chain of 5-10, preferably 6 carbon atoms.

7. A method according to one or more of claims 1-6, **characterized in that** as the gel matrix in the immobilization zone, NHS-activated Sepharose is used.

8. A method according to one or more of claims 1-7, **characterized in that** the analyte is a bloodgroup antigen present on erythrocytes.

9. A method according to one or more of claims 1-8, **characterized in that** the analyte is an antibody binding to a bloodgroup antigen.

10. A method according to one or more of claims 1-9, **characterized in that** a cross-test is carried out **in that** erythrocytes of unknown bloodgroup antigen composition and antibodies of unknown bloodgroup specificity are used.

11. A test kit suitable for use in a method for determining an analyte in a sample, the analyte being a bloodgroup antigen present on erythrocytes or an antibody binding to such a bloodgroup antigen, comprising:
(i) a reagent containing an analyte-binding partner which, in the case where the analyte is a bloodgroup antigen present on erythrocytes, is an antibody capable of binding to the bloodgroup antigen, and in the case where the analyte is an antibody binding to a bloodgroup antigen, is the bloodgroup antigen present on erythrocytes,
(ii) a reaction vessel comprising an incubation zone, an immobilization zone and a collection zone, and
(iii) a separation medium having a density lower than the density of erythrocytes but higher than the density of an antibody-containing fluid,
wherein the immobilization zone comprises a gel matrix carrying IgM-binding antibody or antibody fragment, bound to the gel matrix by direct covalent binding or via a spacer-arm, and the gel matrix optionally further carries complement-binding antibody or antibody fragment, bound to the gel matrix by direct covalent binding or via a spacer-arm.

## Patentansprüche

1. Verfahren zur Bestimmung eines Analyten, der aus einem auf Erythrocyten vorhandenen Blutgruppenantigen und einem Antikörper, der an ein solches Blutgruppenantigen bindet, ausgewählt wird, in einer Probe,
umfassend Behandeln der Probe in einer Inkubationszone eines Reaktionsgefäßes mit einem Reagenz, das einen Analyt-bindenden Partner enthält, wobei der Analyt-bindende Partner, wenn der Analyt ein auf Erythrocyten vorhandenes Blutgruppenantigen ist, ein Antikörper ist, der zur Bindung an das Blutgruppenantigen fähig ist, und wenn der Analyt ein Antikörper, der an ein Blutgruppenantigen bindet, ist, das auf Erythrocyten vorhandene Blutgruppenantigen ist, wobei, wenn die Probe den Analyten enthält, in der Inkubationszone entweder ein Komplex aus auf Erythrocyten vorhandenem Blutgruppenantigen und daran gebundenem Antikörper gebildet wird oder ein Komplex aus auf Erythrocyten vorhandenem Blutgruppenantigen und daran gebundenem Antikörper gebildet wird wie auch ein Komplex aus Komplementfaktoren, die an diese Erythrocyten gebunden sind, gebildet wird, wenn der Antikörper Komplementbindend ist,
außerdem umfassend Abtrennung von Erythrocyten, komplexiert oder nicht, von nicht-gebundenen Antikörpern unter Verwendung eines Trennmediums, das sich im Reaktionsgefäß unter der Inkubationszone befindet, mit einer Dichte, die höher ist als die des Fluids, das die Antikörper enthält, aber niedriger ist als die Dichte von Erythrocyten, wodurch Erythrocyten durch das Trennmedium gehen und nicht-gebundene Antikörper in der Inkubationszone zurückbleiben,
Abtrennen von komplexierten Erythrocyten von nicht-komplexierten Erythrocyten durch Bindung von komplexierten Erythrocyten in einer Immobilisierungszone des Reaktionsgefäßes,
Ausbringen von nicht-komplexierten Erythrocyten in eine Sammelzone des Reaktionsgefäßes und
Detektieren von Erythrocyten in der Immobilisierungszone und/oder Sammelzone,
wobei die Immobilisierungszone eine Gelmatrix umfasst, die einen IgM-bindenden Antikörper oder ein IgM-bindendes Antikörperfragment trägt, der/das durch direkte kovalente Bindung oder über einen Spacerarm an die Gelmatrix gebunden ist.

2. Verfahren nach Anspruch 1, **dadurch gekennzeichnet, dass** die Probe aus Blut, Blutplasma, Blutserum, einer Blutfraktion oder einem Überstand eines Hybridoms besteht.

3. Verfahren nach einem oder mehreren der Ansprüche 1 bis 2, **dadurch gekennzeichnet, dass** die Gelmatrix außerdem einen Komplement-bindenden Antikörper oder ein Komplement-bindendes Antikörperfragment trägt, der/das durch direkte kovalente Bindung oder über einen Spacerarm an die Gelmatrix gebunden ist.

4. Verfahren nach Anspruch 3, **dadurch gekennzeichnet, dass** der Komplement-bindende Antikörper oder das Komplement-bindende Antikörperfragment ein Aktivierungsprodukt von Komplementfaktor C4 oder Komplementfaktor C3 bindet.

5. Verfahren nach Anspruch 4, **dadurch gekennzeichnet, dass** ein Antikörper, der gegen eine antigene Determinante auf C3b oder C4b, zum Beispiel gegen C3c, C3d oder C3g auf C3b, gerichtet ist, verwendet wird.

6. Verfahren nach einem oder mehreren der Ansprüche 1 bis 5, **dadurch gekennzeichnet, dass** der Spacerarm eine Kette von 5 bis 10, vorzugsweise 6 Kohlenstoffatomen enthält.

7. Verfahren nach einem oder mehreren der Ansprüche 1 bis 6, **dadurch gekennzeichnet, dass** NHS-aktivierte Sepharose als Gelmatrix in der Immobilisierungszone eingesetzt wird.

8. Verfahren nach einem oder mehreren der Ansprüche 1 bis 7, **dadurch gekennzeichnet, dass** der Analyt ein auf Erythrocyten vorhandenes Blutgruppenantigen ist.

9. Verfahren nach einem oder mehreren der Ansprüche 1 bis 8, **dadurch gekennzeichnet, dass** der Analyt ein Antikörper ist, der an ein Blutgruppenantigen bindet.

10. Verfahren nach einem der Ansprüche 1 bis 9, **dadurch gekennzeichnet, dass** ein Kreuzungstest durchgeführt wird, in dem Erythrocyten mit einer unbekannten Blutgruppenantigenzusammensetzung und Antikörper mit unbekannter Blutgruppenspezifität verwendet werden.

11. Testkit, der zur Verwendung in einem Verfahren zur Bestimmung eines Analyten in einer Probe geeignet ist, wobei der Analyt ein auf Erythrocyten vorhandenes Blutgruppenantigen oder ein Antikörper, der an ein solches Blutgruppenantigen bindet, ist, umfassend:
(i) ein Reagenz, das einen Analyt-bindenden Partner enthält, der, wenn der Analyt ein auf Erythrocyten vorhandenes Blutgruppenantigen ist, ein Antikörper ist, der zur Bindung an das Blutgruppenantigen fähig ist, und wenn der Analyt ein Antikörper, der an ein Blutgruppenantigen bindet, ist, das auf Blutgruppen vorhandene Blutgruppenantigen ist,
(ii) ein Reaktionsgefäß, das eine Inkubationszone, eine Immobilisierungszone und eine Sammelzone umfasst, und
(iii) ein Trennmedium, das eine Dichte hat, die niedriger ist als die Dichte von Erythrocyten, aber höher ist als die Dichte eines Antikörper-enthaltenden Fluids,
wobei die Immobilisierungszone eine Gelmatrix umfasst, die einen IgM-bindenden Antikörper oder ein IgM-bindendes Antikörperfragment umfasst, der das durch direkte kovalente Bindung oder über einen Spacerarm an die Gelmatrix gebunden ist, und wobei die Gelmatrix gegebenenfalls außerdem einen Komplement-bindenden Antikörper oder ein Komplement-bindendes Antikörperfragment trägt, der das durch direkte kovalente Bindung oder über eine Spacerarm an die Gelmatrix gebunden ist.

## Revendications

1. Procédé pour déterminer dans un échantillon un analyte choisi parmi un antigène de groupe sanguin présent sur des érythrocytes ou un anticorps liant un tel antigène de groupe sanguin,
comprenant le traitement de l'échantillon dans une zone d'incubation d'un récipient réactionnel avec un réactif contenant un partenaire liant l'analyte, lequel partenaire liant l'analyte, dans le cas où l'analyte est un antigène de groupe sanguin présent sur des érythrocytes, est un anticorps capable de lier l'antigène de groupe sanguin, et dans le cas où l'analyte est un anticorps liant un antigène de groupe sanguin, est l'antigène de groupe sanguin présent sur des érythrocytes, où, dans la zone d'incubation, si l'échantillon contient l'analyte, un complexe d'antigène de groupe sanguin présent sur des érythrocytes et d'anticorps lié à celui-ci est formé, ou un complexe d'antigène de groupe sanguin présent sur des érythrocytes et d'anticorps lié à celui-ci est formé, ainsi qu'un complexe de facteurs du complément liés à ces érythrocytes si l'anticorps lie le complément,
comprenant en outre la séparation des érythrocytes, complexés ou pas, d'avec les anticorps non liés au moyen d'un milieu de séparation, situé dans le récipient réactionnel sous la zone d'incubation, d'une densité supérieure à celle du fluide contenant les anticorps mais inférieure à la densité des érythrocytes, de sorte que les érythrocytes traversent le milieu de séparation et les anticorps non liés restent dans la zone d'incubation,
la séparation des érythrocytes complexés d'avec les érythrocytes non complexés par liaison des érythrocytes complexés dans une zone d'immobilisation du récipient réactionnel,
l'évacuation des érythrocytes non complexés dans une zone de collecte du récipient réactionnel, et
la détection des érythrocytes dans la zone d'immobilisation et/ou la zone de collecte,
où ladite zone d'immobilisation comprend une matrice de gel portant un anticorps ou fragment d'anticorps liant les IgM, lié à la matrice de gel par liaison covalente directe ou par le biais d'un bras espaceur.

2. Procédé selon la revendication 1 **caractérisé en ce que** l'échantillon consiste en sang, plasma sanguin, sérum sanguin, une fraction sanguine ou un surnageant d'un hybridome.

3. Procédé selon une ou plusieurs des revendications 1 - 2 **caractérisé en ce que** la matrice de gel porte en outre un anticorps ou fragment d'anticorps liant le complément, lié à la matrice de gel par liaison covalente directe ou par le biais d'un bras espaceur.

4. Procédé selon la revendication 3 **caractérisé en ce que** l'anticorps ou fragment d'anticorps liant le complément lie un produit d'activation du facteur du complément C4 ou du facteur du complément C3.

5. Procédé selon la revendication 4 **caractérisé en ce qu'**un anticorps dirigé contre un déterminant antigénique sur C3b ou C4b, par exemple contre C3c, C3d ou C3g sur C3b est utilisé.

6. Procédé selon une ou plusieurs des revendications 1 - 5 **caractérisé en ce que** le bras espaceur contient une chaîne de 5 à 10, de préférence 6 atomes de carbone.

7. Procédé selon une ou plusieurs des revendications 1 - 6 **caractérisé en ce que** du Sepharose activé par NHS est utilisé comme matrice de gel dans la zone d'immobilisation.

8. Procédé selon une ou plusieurs des revendications 1 - 7 **caractérisé en ce que** l'analyte est un antigène de groupe sanguin présent sur des érythrocytes.

9. Procédé selon une ou plusieurs des revendications 1 - 8 **caractérisé en ce que** l'analyte est un anticorps liant un antigène de groupe sanguin.

10. Procédé selon une ou plusieurs des revendications 1 - 9 **caractérisé en ce qu'**un test croisé dans lequel des érythrocytes de composition d'antigènes de groupe sanguin inconnue et des anticorps de spécificité de groupe sanguin inconnue sont utilisés est réalisé.

11. Kit de test qui convient pour être utilisé dans un procédé pour déterminer un analyte dans un échantillon, l'analyte étant un antigène de groupe sanguin présent sur des érythrocytes ou un anticorps liant un tel antigène du groupe sanguin, comprenant :
(i) un réactif contenant un partenaire liant l'analyte qui, dans le cas où l'analyte est un antigène de groupe sanguin présent sur des érythrocytes, est un anticorps capable de lier l'antigène de groupe sanguin, et dans le cas où l'analyte est un anticorps liant un antigène de groupe sanguin, est l'antigène de groupe sanguin présent sur des érythrocytes,
(ii) un récipient réactionnel comprenant une zone d'incubation, une zone d'immobilisation et une zone de collecte, et
(iii) un milieu de séparation ayant une densité inférieure à la densité des érythrocytes mais supérieure à la densité d'un fluide contenant des anticorps,
où la zone d'immobilisation comprend une matrice de gel portant un anticorps ou fragment d'anticorps liant les IgM, lié à la matrice de gel par liaison covalente directe ou par le biais d'un bras espaceur, et la matrice de gel porte en outre éventuellement un anticorps ou fragment d'anticorps liant le complément, lié à la matrice de gel par liaison covalente directe ou par le biais d'un bras espaceur.
